(19) Europäisches Patentamt
European Patent Office
Office européen des brevets

(11) Veröffentlichungsnummer: **0 129 192**
**A1**

# (12) EUROPÄISCHE PATENTANMELDUNG

(21) Anmeldenummer: 84106722.6

(22) Anmeldetag: 13.06.84

(51) Int. Cl.³: C 07 C 127/19
C 07 C 125/065, C 07 C 155/02
C 07 C 103/34, C 07 C 103/737
C 07 C 87/64, C 07 C 91/28
C 07 C 97/10, C 07 D 295/20

(30) Priorität: 15.06.83 DE 3321582

(43) Veröffentlichungstag der Anmeldung:
27.12.84 Patentblatt 84/52

(84) Benannte Vertragsstaaten:
BE CH DE FR GB IT LI NL

(71) Anmelder: BASF Aktiengesellschaft
Carl-Bosch-Strasse 38
D-6700 Ludwigshafen(DE)

(72) Erfinder: Schirmer, Ulrich, Dr.
Berghalde 79
D-6900 Heidelberg(DE)

(72) Erfinder: Goetz, Norbert, Dr.
Schoefferstrasse 25
D-6520 Worms 1(DE)

(72) Erfinder: Wuerzer, Bruno, Dr. Dipl.-Landwirt
Ruedigerstrasse 13
D-6701 Otterstadt(DE)

## (54) Anilinderivate, Verfahren zu ihrer Herstellung und ihre Verwendung zur Bekämpfung unerwünschten Pflanzenwuchses.

(57) Die Erfindung betrifft Anilinderivate der Formel

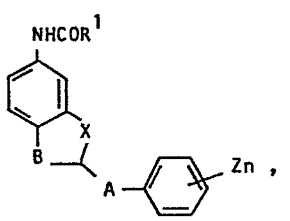

in der

R¹ Alkoxy, Alkylthio oder gegebenenfalls substituiertes Cycloalkoxy oder den Rest $-NR^2R^3$,

X $-CH_2-$, $-CH(OH)-$ oder $-CO-$,

A eine gegebenenfalls substituierte Alkylenkette,

B gegebenenfalls substituiertes Methylen oder Dimethylen,

Z Wasserstoff, Halogen, Alkyl, Alkoxy, Halogenalkyl oder Halogen-alkoxy und

n die Zahlen 1, 2 oder 3 bedeuten,

Verfahren zu ihrer Herstellung und ihre Verwendung zur Bekämpfung unerwünschten Pflanzenwuchses.

EP 0 129 192 A1

Croydon Printing Company Ltd

Anilinderivate, Verfahren zu ihrer Herstellung und ihre Verwendung zur Bekämpfung unerwünschten Pflanzenwuchses

Die vorliegende Erfindung betrifft Anilinderivate, Verfahren zu ihrer Herstellung, Herbizide, die diese Verbindungen als Wirkstoffe enthalten, sowie ein Verfahren zur Bekämpfung unerwünschten Pflanzenwuchses mit diesen Verbindungen.

Es ist bekannt, daß N-Methoxy-N-methyl-N'-phenyl-harnstoffe herbizid wirksam sind.

Es wurde gefunden, daß Anilinderivate der Formel

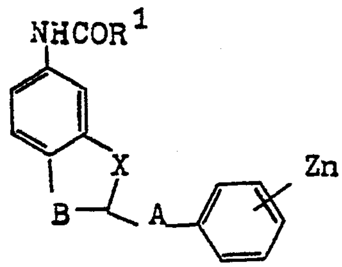
(I),

in der

$R^1$ $C_1$-$C_4$-Alkoxy, $C_1$-$C_4$-Alkylthio oder gegebenenfalls durch Halogen oder $C_1$-$C_5$-Alkoxy substituiertes $C_1$-$C_4$-Alkyl, $C_3$-$C_6$-Cycloalkyl, $C_3$-$C_6$-Cycloalkoxy oder den Rest $-NR^2R^3$, wobei $R^2$ und $R^3$ unabhängig voneinander Wasserstoff, $C_1$-$C_4$-Alkyl oder $C_1$-$C_4$-Alkoxy oder $C_3$-$C_6$-Cycloalkyl oder $C_3$-$C_6$-Cycloalkoxy bedeuten oder $R^2$ und $R^3$ zusammen eine gegebenenfalls durch Methyl substituierte und gegebenenfalls Sauerstoff als Kettenglied enthaltende Alkylenkette mit bis zu 6 Kohlenstoffatomen bedeuten,

X Methylen, -CH(OH)- oder -CO-,

A eine gegebenenfalls durch Alkyl mit 1 bis 5 Kohlenstoffatomen substituierte Alkylenkette mit 1 bis 5 Kohlenstoffatomen,

B gegebenenfalls durch Alkyl mit 1 bis 3 Kohlenstoffatomen substituiertes Methylen oder Ethylen,

Z Wasserstoff, Halogen, $C_1$-$C_6$-Alkyl, $C_1$-$C_6$-Alkoxy, $C_1$-$C_6$-Halogenalkyl oder $C_1$-$C_6$-Halogenalkoxy und

n die Zahlen 1, 2 oder 3 bedeuten,

eine herbizide Wirkung haben und für eine Reihe von Kulturpflanzen ein hohes Maß an Verträglichkeit besitzen.

Die Substituenten in Formel I können die folgenden Bedeutungen haben:

H/P

$R^1$ bedeutet $C_1$–$C_4$-Alkoxy, $C_1$–$C_4$-Alkylthio oder gegebenenfalls durch Halogen oder $C_1$–$C_5$-Alkoxy substituiertes $C_1$–$C_5$-Alkyl, beispielsweise Methoxy, Ethoxy, n-Propoxy, Isopropoxy, sec-Butoxy, tert.-Butoxy, n-Pentoxy, Methylthio, Ethylthio, n-Propylthio, sec.-Butylthio, tert.-Butylthio, Methyl, Ethyl, n-Propyl, n-Butyl, sec.-Butyl, tert.-Butyl, Isobutyl, n-Pentyl, Chlormethyl, 1,1-Dichlorethyl, Dichlormethyl, Trichlormethyl, Methoxymethyl, 1-Methoxyethyl, 2-Methoxyethyl, $C_3$–$C_6$-Cycloalkyl, beispielsweise Cyclopropyl, Cyclopentyl, Cyclohexyl, $C_3$–$C_6$-Cycloalkoxy, beispielsweise Cyclohexoxy, oder den Rest $-NR^2R^3$, wobei $R^2$ und $R^3$ unabhängig voneinander Wasserstoff, $C_1$–$C_4$-Alkyl oder $C_1$–$C_4$-Alkoxy, beispielsweise Methyl, Ethyl, Isopropyl, n-Butyl, sec.-Butyl, Isobutyl, tert.-Butyl, Methoxy, Ethoxy, $C_3$–$C_6$-Cycloalkyl, beispielsweise Cyclopropyl, Cyclopentyl, Cyclohexyl, oder $C_3$–$C_6$-Cycloalkoxy, beispielsweise Cyclohexoxy, oder $R^2$ und $R^3$ zusammen eine gegebenenfalls durch Methyl substituierte und gegebenenfalls Sauerstoff als Kettenglied enthaltende Alkylenkette mit bis zu 6 Kohlenstoffatomen, beispielsweise Trimethylen, Tetramethylen, 1,4-Dimethyltetramethylen, Pentamethylen, Hexamethylen, 3-Oxapentamethylen, 1-Oxapentamethylen, 1-Oxa-tetramethylen, bedeuten.

A bedeutet eine Alkylenkette mit 1 bis 5 Kohlenstoffatomen, die durch $C_1$–$C_5$-Alkyl, insbesondere durch Methyl, substituiert sein kann, beispielsweise $-CH_2-$, $-CH(CH_3)-$, $-(CH_2)_3-$, $-(CH_2)_2-$, $-CH_2CH(CH_3)CH_2-$, $-(CH_2)_5-$ oder $-CH_2CH(CH_3)(CH_2)_3-$.

B bedeutet gegebenenfalls durch $C_1$–$C_3$-Alkyl, insbesondere durch Methyl, substituiertes Methylen oder Dimethylen, beispielsweise $-CH_2-$, $-CH(CH_3)-$, $-(CH_2)_2-$, $-CH(CH_3)CH_2-$ oder $-CH_2CH(CH_3)-$.

Z bedeutet Wasserstoff, Halogen, beispielsweise Fluor, Chlor, Brom, $C_1$–$C_6$-Alkyl, $C_1$–$C_6$-Alkoxy, $C_1$–$C_6$-Halogenalkyl oder $C_1$–$C_6$-Halogenalkoxy, beispielsweise Methyl, Ethyl, Isopropyl, tert.-Butyl, n-Hexyl, Methoxy, Ethoxy, Isopropoxy, tert.-Butoxy, n-Hexoxy, Trifluormethyl, 1,1,2-Trifluor-2-chlorethoxy, Difluormethoxy. Z steht in ortho-, meta- oder para-Stellung zu A; n ist vorzugsweise 1, kann jedoch auch 2 oder 3 bedeuten, insbesondere, wenn Z für Fluor, Chlor, Brom oder Methyl steht.

Bevorzugte Verbindungen der Formel I sind solche, bei denen $R^1$ den Rest $-N(CH_3)(OCH_3)$, A Methylen, B Methylen oder Dimethylen, X Methylen, Z Wasserstoff, Halogen, insbesondere Chlor, oder $C_1$–$C_4$-Alkyl oder $C_1$–$C_4$-Alkoxy, insbesondere Methyl oder Methoxy, und n 1 bedeuten.

Man erhält die Anilinderivate der Formel I durch Umsetzung von Aminen der Formel

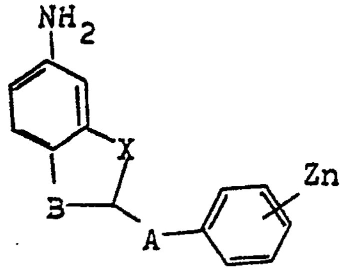 (II),

in der
A, X, B, Z und n die obengenannnten Bedeutungen haben, mit Verbindungen der Formel

$R^1$-CO-X (III),

in der $R^1$ die obengenannten Bedeutungen hat und X eine Abgangsgruppe, vorzugsweise Halogen, wie Chlor oder Brom, oder $R^1$-CO-O-, bedeutet.

Die Umsetzung wird in einem inerten organischen Lösungsmittel durchgeführt. Geeignet sind Ether, wie Tetrahydrofuran, Dimethoxyethan, Diethylether, Methyl-tert.-butyl-ether, Ester wie Essigsäureethylester, gegebenenfalls chlorierte aliphatische oder aromatische Kohlenwasserstoffe, wie Toluol, Dichlormethan, Pyridin. Auch Gemische dieser Lösungsmttel können verwendet werden. Die Menge an Lösungsmittel, bezogen auf Amin der Formel II beträgt 100 bis 5 000 Gew.%.

Zweckmäßigerweise wird die Umsetzung in Gegenwart eines Säureakzeptors durchgeführt. In Betracht kommen Alkalihydroxide, Alkalicarbonate, Alkalihydrogencarbonate, Erdalkalihydroxide, Erdalkalicarbonate, Erdalkalihydrogencarbonate, Erdalkalioxide oder Amine, beispielsweise Natriumhydrogencarbonat, Kaliumcarbonat, Triethylamin, Pyridin, N,N-Dimethylanilin, N,N-Dimethyl-N-cyclohexylamin, Chinolin. Die Menge an Säureakzeptor beträgt 1 bis 4 Mol, bezogen auf 1 Mol Verbindung der Formel III.

Die Ausgangsstoffe der Formeln II und III werden bevorzugt in äquimolarem Verhältnis miteinander umgesetzt. Die Reaktionstemperatur liegt zwischen 0 und 80°C, vorzugsweise zwischen 20 und 30°C.

Die Amine der Formel II sind neu. Sie werden nach bekannten Verfahren durch Aldolkondensation und anschließende Hydrierung erhalten. Man setzt beispielsweise gegebenenfalls substituierte Nitro-alpha-indanone oder Nitro-alpha-tetralone mit gegebenenfalls substituierten Benzaldehyden zu

Nitrochalkonen um (R. J. Murray, N. A. Cromwell, J. Org. Chem. 41, 3540 - 3545 (1976)).

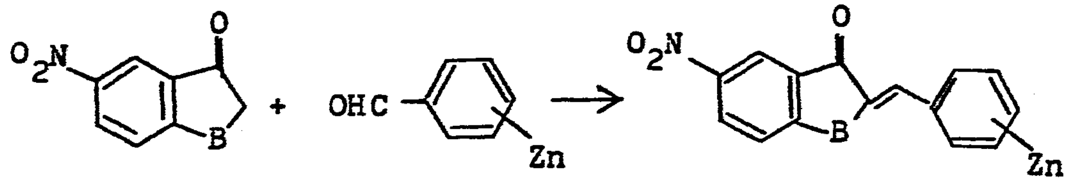

und hydriert die so erhaltenen Nitrochalkone in an sich bekannter Weise in einem Essigsäure/Schwefelsäure-Gemisch (wenn X = Methylen) bzw. in einem organischen Lösungsmittel, wie Methanol oder Tetrahydrofuran (wenn X = -CO- oder -CH(OH)-), in Gegenwart von Palladium auf Kohle oder Raney-Nickel bei Temperaturen zwischen 0 und 150°C, vorzugsweise zwischen 20 und 80°C, gegebenenfalls unter Druck. B, Y, Z und n haben dabei die obengenannten Bedeutungen.

Amine der Formel II, bei denen A $-CH_2CH(CH_3)CH_2-$ oder $-(CH_2)_3-$ bedeutet, werden dadurch erhalten, daß man gegebenenfalls substituierte Nitroalpha-Tetralone oder Nitroalpha-Indanone mit gegebenenfalls substituierten Zimtaldehyden nach folgendem Schema kondensiert

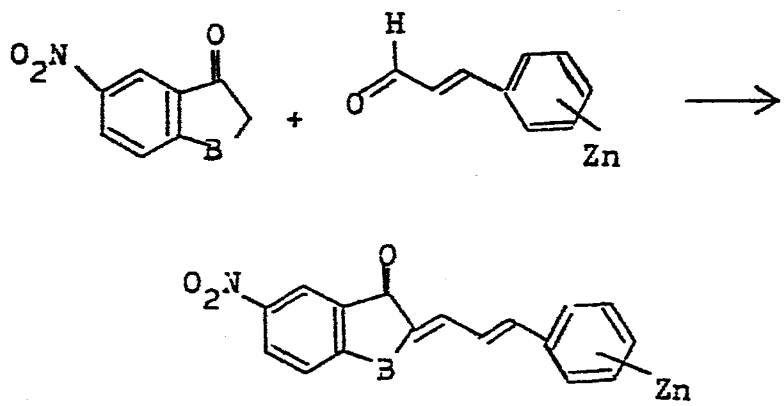

und anschließend hydriert.

Die Wasserstoffaufnahme bei der Hydrierung der vorgenannten Nitrophenylchalkone erfolgt stufenweise und kann beim Arbeiten in Tetrahydrofuran entweder nach Erreichen der gesättigten Anilinoketon-Stufe (X = -CO-) abgebrochen oder bis zur Anilinocarbinolstufe (X = -CH(OH)-) weitergeführt werden bzw. beim Arbeiten in Essigsäure/Schwefelsäure-Gemisch direkt bis zur Anilino-Kohlenwasserstoffstufe (X= $-CH_2-$) durchgeführt werden.

Die Hydrierung erfolgt in an sich bekannter Weise in Gegenwart von Raney-Nickel oder vorzugsweise von Palladium auf Aktivkohle. In diesem Fall gelingt die Hydrierung zum gesättigten Anilinoketon (X = -CO-)

bereits bei Temperaturen von 0 bis 30°C, vorzugsweise bei 25°C, und bei einem Druck von 1,0 bis 20 bar, bevorzugt bei 1,1 bar. Verlängerung der Reaktionsdauer, Erhöhung der Hydriertemperatur und/oder Druckerhöhung bis zu 50 bar führt zur Reduktion der Ketogruppe zur Carbinolgruppe (X = -CH(OH)-) (Houben-Weyl, Methoden der Org. Chemie, Bd. 4/1c, S. 13 ff, Georg-Thieme-Verlag, Stuttgart, 1980).

Die folgenden Beispiele veranschaulichen die Synthese der Amine der Formel II:

Beispiel A

72 g 7-Nitro-α-tetralon, 40 g Benzaldehyd und 10 g Borsäure werden unter Wasserauskreisung in 500 ml Xylol 18 Stunden lang gekocht, die noch heiße Lösung in Toluol eingerührt und nach dem Erkalten abgesaugt. Man erhält 84,9 g (Ausbeute: 81 %) 7-Nitrobenzyliden-α-tetralon (Fp. 164 - 166°C)

84 g 7-Nitro-benzyliden-α-tetralon werden in 30 g Schwefelsäure und 1000 ml Eisessig gelöst, mit 5 g Palladium/Aktivkohle (10 %ig) versetzt und bei einem Druck von 1,1 bar bei 70°C bis zur Aufnahme von 43,5 l Wasserstoff hydriert. Danach wird filtriert, eingeengt, mit 10 %iger Natronlauge alkalisch gemacht und mit Diethylether ausgeschüttelt. Nach dem Trocknen der organischen Phase über Natriumsulfat, Filtrieren und Destillieren erhält man 52,7 g (Ausbeute: 74 %) Amin der Formel

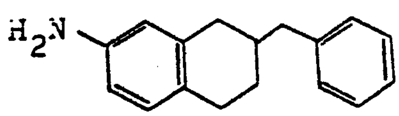

vom Siedepunkt 162 - 164°C/0,7 mbar

Beispiel B

118 g 7-Nitro-(4'-Chlorbenzyliden)-α-tetralon werden in 1 000 ml Tetrahydrofuran gelöst und bei 20 bis 25°C in Gegenwart von 4 g 10 %iger Palladium-Tierkohle und einem Druck von 1,1 bar bis zur Aufnahme von 33,5 l Wasserstoff hydriert. Nach dem Trocknen über Natriumsulfat wird filtiert und eingeengt. Der verbliebene Rückstand wird aus Acetonitril umkristallisiert. Man erhält 51,7 g (Ausbeute: 48 %) Amin der Formel:

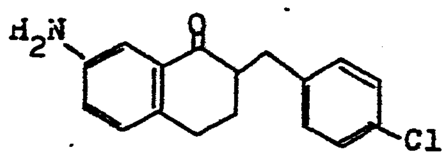

vom Schmelzpunkt 168 - 170°C.

Entsprechend können folgende Amine der Formel II hergestellt werden:

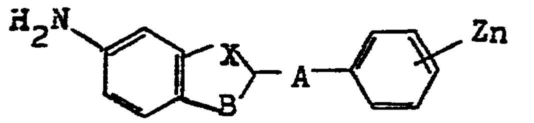

| A | B | X | Zn | Fp[°C] |
|---|---|---|---|---|
| $-CH_2-$ | $-CH_2-CH_2-$ | $-CO-$ | H | 98-100 |
| $-CH_2-$ | $-CH_2-CH_2-$ | $-CH(OH)-$ | H | 120-125 |
| $-CH_2-$ | $-CH_2-$ | $-CO-$ | H | 149-151 |
| $-CH_2-$ | $-CH_2-$ | $-CH_2-$ | H | 52-54 |
| $-CH_2-$ | $-CH_2-CH_2-$ | $-CH_2-$ | 4-Cl | 71-73 |
| $-CH_2-$ | $-CH_2-CH_2-$ | $-CH_2-$ | 4-F | 71-75 |
| $-CH_2-$ | $-CH_2-CH_2-$ | $-CH_2-$ | $4-CH_3$ | 90-92 |
| $-CH_2-$ | $-CH_2-CH_2-$ | $-CH_2-$ | $4-OCH_3$ | 83-86 |
| $-CH_2-$ | $-CH_2-CH_2-$ | $-CH_2-$ | $3,4-(CH_3)_2$ | |
| $-CH_2-$ | $-CH_2-CH_2-$ | $-CH_2-$ | $4-t-C_4H_9$ | |
| $-CH_2-$ | $-CH_2-CH_2-$ | $-CH_2-$ | $3-CF_3$ | |
| $-CH_2-$ | $-CH_2-CH_2-$ | $-CH_2-$ | $4-OCHF_2$ | |
| $-CH_2-$ | $-CH_2-CH_2-$ | $-CH_2-$ | $4-O-n-C_6H_{13}$ | |
| $-CH_2-CH_2-CH_2-$ | $-CH_2-CH_2-$ | $-CH_2-$ | H | 48-50 |
| $-CH_2-CH_2-CH_2-$ | $-CH_2-CH_2-$ | $-CO-$ | H | 55-58 |
| $-CH_2-$ | $-CH_2-CH_2-$ | $-CO-$ | 4-F | |
| $-CH_2-$ | $-CH_2-CH_2-$ | $-CO-$ | $4-CH_3$ | 113-117 |
| $-CH_2-$ | $-CH_2-CH_2-$ | $-CO-$ | $4-OCH_3$ | 135-138 |
| $-CH_2-$ | $-CH_2-CH_2-$ | $-CO-$ | $3,4-Cl_2$ | |
| $-CH_2-$ | $-CH_2-CH_2-$ | $-CO-$ | 3-Cl | |
| $-CH_2-CH(CH_3)-CH_2-$ | $-CH_2-CH_2-$ | $-CH_2-$ | H | |
| $-(CH_2)_5$ | $-CH_2-CH_2-$ | $-CH_2-$ | H | |

Folgendes Beispiel erläutert die Herstellung der Anilinderivate der Formel I.

Beispiel 1

10,7 g 2-Benzyl-7-amino-1,2,3,4-tetrahydronaphthalen (Beispiel A) werden in 150 ml Tetrahydrofuran gelöst, mit 4,6 g Natriumhydrogencarbonat versetzt und unter gutem Rühren bei 20 bis 25°C mit 5,6 g N-Methoxy-N-methylcarbaminsäurechlorid zur Reaktion gebracht. Nach 12stündigem Rühren wird filtriert, eingeengt, mit Petrolether verrieben und abgesaugt. Man erhält 11,6 g (Ausbeute 79,5 %) Anilinderivat der Formel

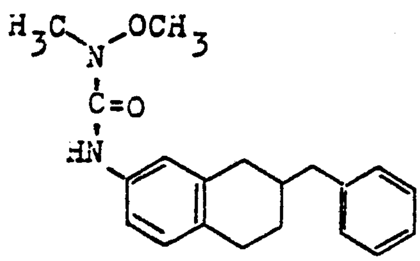

vom Schmelzpunkt 118 - 120°C.

Entsprechend können folgende Verbindungen der Formel I hergestellt werden:

| | $R^1$ | A | B | X | Zn | Fp [°C] |
|---|---|---|---|---|---|---|
| 2 | $C_2H_5$ | $-CH_2-$ | $-CH_2-CH_2-$ | $-CH_2-$ | H | 85 - 87 |
| 3 | $N(CH_3)_2$ | $-CH_2-$ | $-CH_2-CH_2-$ | $-CH_2-$ | H | 142 - 144 |
| 4 | $SCH_3$ | $-CH_2-$ | $-CH_2-CH_2-$ | $-CH_2-$ | H | 85 - 89 |
| 5 | 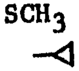 | $-CH_2-$ | $-CH_2-CH_2-$ | $-CH_2-$ | H | 162 - 165 |
| 6 | $-NHCH_3$ | $-CH_2-$ | $-CH_2-CH_2-$ | $-CH_2-$ | H | |
| 7 | $N(OC_2H_5)C_2H_5$ | $-CH_2-$ | $-CH_2-CH_2-$ | $-CH_2-$ | H | |
| 8 | 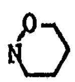 | $-CH_2-$ | $-CH_2-CH_2-$ | $-CH_2-$ | H | |
| 9 | $t-C_4H_9$ | $-CH_2-$ | $-CH_2-CH_2-$ | $-CH_2-$ | H | |
| 10 | 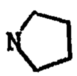 | $-CH_2-$ | $-CH_2-CH_2-$ | $-CH_2-$ | H | |
| 11 | 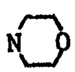 | $-CH_2-$ | $-CH_2-CH_2-$ | $-CH_2-$ | H | |
| 12 | $-CCl_2CH_3$ | $-CH_2-$ | $-CH_2-CH_2-$ | $-CH_2-$ | H | |
| 13 | $-CH_2OCH_3$ | $-CH_2-$ | $-CH_2-CH_2-$ | $-CH_2-$ | H | |

| | $R^1$ | A | B | X | Zn | Fp [°C] |
|---|---|---|---|---|---|---|
| 14 | $O-t-C_4H_9$ | $-CH_2-$ | $-CH_2-CH_2-$ | $-CH_2-$ | H | |
| 15 | $S-n-C_3H_7$ | $-CH_2-$ | $-CH_2-CH_2-$ | $-CH_2-$ | H | |
| 16 | $N(OCH_3)CH_3$ | $-CH_2-$ | $-CH_2-CH_2-$ | $-CH_2-$ | 4-Cl | 102 - 103 |
| 17 | $C_2H_5$ | $-CH_2-$ | $-CH_2-CH_2-$ | $-CH_2-$ | 4-Cl | 121 - 122 |
| 18 | $N(OCH_3)CH_3$ | $-CH_2-$ | $-CH_2-CH_2-$ | $-CH_2-$ | 4-F | 111 - 114 |
| 19 | $C_2H_5$ | $-CH_2-$ | $-CH_2-CH_2-$ | $-CH_2-$ | $4-t-C_4H_9$ | |
| 20 | $N(CH_3)_2$ | $-CH_2-$ | $-CH_2-CH_2-$ | $-CH_2-$ | 4-F | 145 - 148 |
| 21 | $N(CH_3)_2$ | $-CH_2-$ | $-CH_2-CH_2-$ | $-CH_2-$ | 4-Cl | |
| 22 | $N(CH_3)_2$ | $-CH_2-$ | $-CH_2-CH_2-$ | $-CH_2-$ | $4-CH_3$ | |
| 23 | $N(CH_3)_2$ | $-CH_2-$ | $-CH_2-CH_2-$ | $-CH_2-$ | $4-OCH_3$ | |
| 24 | $N(OCH_3)CH_3$ | $-CH_2-$ | $-CH_2-CH_2-$ | $-CH_2-$ | $4-OCH_3$ | 106 - 108 |
| 25 | $C_2H_5$ | $-CH_2-$ | $-CH_2-CH_2-$ | $-CH_2-$ | $4-OCH_3$ | 139 - 141 |
| 26 | $C_2H_5$ | $-CH_2-$ | $-CH_2-CH_2-$ | $-CH_2-$ | $4-CH_3$ | |
| 27 | $N(OCH_3)CH_3$ | $-CH_2-$ | $-CH_2-CH_2-$ | $-CH_2-$ | $4-CH_3$ | 122 - 124 |
| 28 | $N(OCH_3)CH_3$ | $-CH_2-$ | $-CH_2-CH_2-$ | $-CH_2-$ | $3,4-(CH_3)_2$ | |
| 29 | $N(OCH_3)CH_3$ | $-CH_2-$ | $-CH_2-CH_2-$ | $-CH_2-$ | $4-t-C_4H_9$ | |
| 30 | $N(OCH_3)CH_3$ | $-CH_2-$ | $-CH_2-CH_2-$ | $-CH_2-$ | $3-CF_3$ | |
| 31 | $N(OCH_3)CH_3$ | $-CH_2-$ | $-CH_2-CH_2-$ | $-CH_2-$ | $4-OCHF_2$ | |
| 32 | $N(OCH_3)CH_3$ | $-CH_2-$ | $-CH_2-CH_2-$ | $-CH_2-$ | $4-O-n-C_6H_{13}$ | |
| 33 | $N(OCH_3)CH_3$ | $-CH_2-$ | $-CH_2-CH_2-$ | -CO- | 4-Cl | 129 - 131 |
| 34 | $C_2H_5$ | $-CH_2-$ | $-CH_2-CH_2-$ | -CO- | 4-Cl | 110 - 112 |
| 35 | $N(CH_3)_2$ | $-CH_2-$ | $-CH_2-CH_2-$ | -CO- | 4-Cl | |

| | $R^1$ | A | B | X | Zn | Fp [°C] |
|---|---|---|---|---|---|---|
| 36 | $N(CH_3)_2$ | $-CH_2-$ | $-CH_2-CH_2-$ | $-CO-$ | 4-F | |
| 37 | $C_2H_5$ | $-CH_2-$ | $-CH_2-CH_2-$ | $-CO-$ | $4-CH_3$ | 108 - 110 |
| 38 | $N(CH_3)_2$ | $-CH_2-$ | $-CH_2-CH_2-$ | $-CO-$ | $4-OCH_3$ | |
| 39 | $N(CH_3)_2$ | $-CH_2-$ | $-CH_2-CH_2-$ | $-CO-$ | H | |
| 40 | $C_2H_5$ | $-CH_2-$ | $-CH_2-CH_2-$ | $-CO-$ | H | 130 - 132 |
| 41 | $C_2H_5$ | $-CH_2-$ | $-CH_2-CH_2-$ | $-CH_2-$ | 4-F | 117 - 119 |
| 42 | $C_2H_5$ | $-CH_2-$ | $-CH_2-CH_2-$ | $-CH_2-$ | $3,4-(CH_3)_2$ | |
| 43 | $C_2H_5$ | $-CH_2-$ | $-CH_2-CH_2-$ | $-CH_2-$ | $3-CF_3$ | |
| 44 | $N(OCH_3)CH_3$ | $-CH_2-$ | $-CH_2-CH_2-$ | $-CH_2-$ | H | |
| 45 | $N(OCH_3)CH_3$ | $-CH_2-$ | $-CH_2-CH_2-$ | -CH(OH) | H | Öl |
| 46 | $N(OCH_3)CH_3$ | $-CH_2-$ | $-CH_2-CH_2-$ | $-CO-$ | 4-F | |
| 47 | $N(OCH_3)CH_3$ | $-CH_2-$ | $-CH_2-CH_2-$ | $-CO-$ | $4-CH_3$ | 129 - 131 |
| 48 | $N(OCH_3)CH_3$ | $-CH_2-$ | $-CH_2-CH_2-$ | $-CO-$ | $4-OCH_3$ | 118 - 120 |
| 49 | $N(OCH_3)CH_3$ | $-CH_2-$ | $-CH_2-CH_2-$ | $-CO-$ | $3,4-Cl_2$ | |
| 50 | $N(OCH_3)CH_3$ | $-CH_2-$ | $-CH_2-CH_2-$ | $-CO-$ | 3-Cl | |
| 51 | $N(OCH_3)CH_3$ | $-CH_2-$ | $-CH_2-$ | $-CO-$ | H | 111 - 113 |
| 52 | $N(OCH_3)CH_3$ | $-CH_2CH_2CH_2-$ | $-CH_2-CH_2-$ | $-CO-$ | H | Öl |
| 53 | $C_2H_5$ | $-CH_2CH_2CH_2-$ | $-CH_2-CH_2-$ | $-CO-$ | H | |
| 54 | $N(OCH_3)CH_3$ | $-CH_2-CH_2-CH_2-$ | $-CH_2-$ | $-CH_2-$ | H | |
| 55 | $C_2H_5$ | $-CH_2-CH_2-CH_2-$ | $-CH_2-$ | $-CH_2-$ | H | |
| 56 | $N(CH_3)_2$ | $-CH_2-CH_2-CH_2-$ | $-CH_2-$ | $-CH_2-$ | H | |

| | $R^1$ | A | B | X | Zn | Fp °C |
|---|---|---|---|---|---|---|
| 57 | $N(CH_3)_2$ | $-CH_2-$ | $-CH_2-$ | $-CH_2-$ | H | |
| 58 | $C_2H_5$ | $-CH_2-$ | $-CH_2-$ | $-CH_2-$ | H | |
| 59 | $N(OCH_3)CH_3$ | $-CH_2-$ | $-CH_2-$ | $-CH_2-$ | H | 70 - 72 |
| 60 | $N(OCH_3)CH_3$ | $-CH_2-CH(CH_3)-CH_2-$ | $-CH_2-CH_2-$ | $-CH_2-$ | H | |
| 61 | $C_2H_5$ | $-CH_2-CH(CH_3)-CH_2-$ | $-CH_2-CH_2-$ | $-CH_2-$ | H | |
| 62 | $N(CH_3)_2$ | $-CH_2-CH(CH_3)-CH_2-$ | $-CH_2-CH_2-$ | $-CH_2-$ | H | |
| 63 | $N(CH_3)_2$ | $-CH_2CH_2CH_2-$ | $-CH_2-CH_2-$ | $-CH_2-$ | H | |
| 64 | $N(OCH_3)CH_3$ | $-CH_2CH_2CH_2-$ | $-CH_2-CH_2-$ | $-CH_2-$ | H | 65 - 67 |
| 65 | $C_2H_5$ | $-CH_2CH_2CH_2-$ | $-CH_2-CH_2-$ | $-CH_2-$ | H | 81 |
| 66 | $C_2H_5$ | $-(CH_2)_5-$ | $-CH_2-CH_2-$ | $-CH_2-$ | H | |
| 67 | $N(OCH_3)CH_3$ | $-(CH_2)_5-$ | $-CH_2-CH_2-$ | $-CH_2-$ | H | |
| 68 | $N(CH_3)_2$ | $-(CH_2)_5-$ | $-CH_2-CH_2-$ | $-CH_2-$ | H | |
| 69 | $OCH_3$ | $-CH_2-$ | $-CH_2-CH_2-$ | $-CH_2-$ | 4-$OCH_3$ | 147 - 149 |
| 70 | $N(OCH_3)CH_3$ | $-CH_2-$ | $-CH_2-CH_2-$ | -CO- | H | 108 - 110 |
| 71 | $N(CH_3)_2$ | $-CH_2-$ | $-CH_2-$ | -CO- | H | 168 - 170 |
| 72 | $C_2H_5$ | $-CH_2-$ | $-CH_2-CH_2-$ | -CH(OH)- | H | 126 - 128 |

Die Verbindungen der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind:

I. Man vermischt 90 Gewichtsteile der Verbindung Nr. 1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile der Verbindung Nr. 25 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteile des Anlagerungsproduktes von 40 Mol Ethylenoxid an 1 Mol Ricinusöl besteht. Durch Eingießen und feines Verteilen der Lösung in

100 000 Gewichtsteilen Wasser erhält man eine wäßrige Dispersion, die 0,02 Gewichtsprozent des Wirkstoffs enthält.

IV. 20 Gewichtsteile der Verbindung Nr. 3 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 20 Gewichtsteile der Verbindung Nr. 17 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 3 Gewichtsteile der Verbindung Nr. 33 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile der Verbindung Nr. 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile der Verbindung Nr. 1 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation der Wirkstoffe bzw. der Mittel kann im Vorauflaufverfahren oder im Nachauflaufverfahren erfolgen. Sind die Wirkstoffe für die Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Bekämpfungsziel und Wachstumsstadium 0,05 bis 5 kg/ha und mehr, vorzugsweise 0,1 bis 3 kg/ha.

Die herbizide Wirkung von Verbindungen der Formel I wird durch Gewächshausversuche gezeigt:

Als Kulturgefäße dienen Plastikblumentöpfe mit 300 $cm^3$ Inhalt und lehmigem Sand mit etwa 1,5 % Humus als Substrat. Die Samen der Testpflanzen werden nach Arten getrennt flach eingesät. Bei Vorauflaufbehandlung werden die Wirkstoffe unmittelbar danach auf die Erdoberfläche aufgebracht. Sie werden hierbei in Wasser als Verteilungsmittel suspendiert oder emulgiert und mittels fein verteilender Düsen gespritzt. Die Aufwandmenge beträgt 3,0 kg Wirkstoff/ha. Nach dem Aufbringen der Mittel werden die Gefäße leicht beregnet, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zwecke der Nachauflaufbehandlung zieht man die Testpflanzen je nach Wuchsform erst bis zu einer Wuchshöhe von 3 bis 15 cm an und behandelt sie danach. Es werden direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Die Aufwandmengen für die Nachauflaufbehandlung variieren je nach Wirkstoff; sie betragen beispielsweise 0,125, 1,0 oder 2,0 kg Wirkstoff/ha. Eine Abdeckung unterbleibt bei der Nachauflaufbehandlung.

Die Versuchsgefäße werden im Gewächshaus aufgestellt, wobei für wärmeliebende Arten wärmere Bereiche (20 bis 35°C) und für solche gemäßigter Klimate 10 bis 20°C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile.

Die in den Gewächshausversuchen verwendeten Pflanzen setzen sich aus folgenden Arten zusammen:

Alopecurus myosuroides (Ackerfuchsschwanz), Amaranthus retroflexus (zurückgekrümmter Fuchsschwanz), Amaranthus spp. (Fuchsschwanzarten), Cassia tora, Chenopodium album (Weißer Gänsefuß), Chrysanthemum spp.

(Wucherblume), Euphorbia geniculata (Südamerik. Wolfsmilchsart), Galium aparine (Klettenlabkraut), Ipomoea spp. (Prunkwindearten), Lamium amplexicaule (Stengelumfassende Taubnessel), Sesbania exaltata (Turibaum), Sida spinosa, Sinapis alba (Weißer Senf), Solanum nigrum (Schwarzer Nachtschatten), Triticum aestivum (Weizen).

Bei Vorauflaufanwendung von 3,0 kg Wirkstoff/ha zeigen beispielsweise die Verbindungen Nr. 1, 2, 3 und 33 eine gute herbizide Aktivität an Sinapis alba.

Bei Nachauflaufanwendung von 0,125 kg Wirkstoff/ha bekämpft beispielsweise Verbindung Nr. 1 breitblättrige unerwünschte Pflanzen. Weizen wird dabei nur zu Anfang gering geschädigt. Eine gute Nachauflaufwirkung gegen unerwünschte Pflanzen zeigt beispielsweise auch Verbindung Nr. 33 mit 1,0 kg Wirkstoff/ha sowie die Verbindungen Nr. 2 und Nr. 17 mit 2,0 kg Wirkstoff/ha.

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwünschter Pflanzen eingesetzt werden.

Zur Verbreiterung des Wirkungsspektrums und zur Erzielung synergistischer Effekte können die Anilinderivate der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate und andere in Betracht.

Außerdem kann es von Nutzen sein, die Verbindungen der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Patentansprüche

1. Anilinderivate der Formel

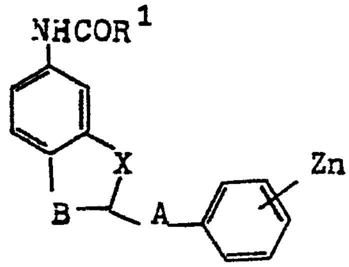 (I),

in der

$R^1$ $C_1$-$C_4$-Alkoxy, $C_1$-$C_4$-Alkylthio oder gegebenenfalls durch Halogen oder $C_1$-$C_5$-Alkoxy substituiertes $C_1$-$C_4$-Alkyl, $C_3$-$C_6$-Cycloalkyl, $C_3$-$C_6$-Cycloalkoxy oder den Rest $-NR^2R^3$, wobei $R^2$ und $R^3$ unabhängig voneinander Wasserstoff, $C_1$-$C_4$-Alkyl oder $C_1$-$C_4$-Alkoxy, $C_3$-$C_6$-Cycloalkyl oder $C_3$-$C_6$-Cycloalkoxy bedeuten oder $R^2$ und $R^3$ zusammen eine gegebenenfalls durch Methyl substituierte und gegebenenfalls Sauerstoff als Kettenglied enthaltende Alkylenkette mit bis zu 6 Kohlenstoffatomen bedeuten,

X $-CH_2-$, $-CH(OH)-$ oder $-CO-$,

A eine gegebenenfalls durch $C_1$-$C_5$-Alkyl substituierte Alkylenkette mit 1 bis 5 Kohlenstoffatomen,

B gegebenenfalls durch $C_1$-$C_3$-Alkyl substituiertes Methylen oder Dimethylen,

Z Wasserstoff, Halogen, $C_1$-$C_6$-Alkyl, $C_1$-$C_6$-Alkoxy, $C_1$-$C_6$-Halogenalkyl oder $C_1$-$C_6$-Halogenalkoxy und

n die Zahlen 1, 2 oder 3 bedeuten.

2. Anilinderivate der Formel I gemäß Anspruch 1, dadurch gekennzeichnet, daß $R^1$ den Rest $-N(CH_3)(OCH_3)$, A Methylen, X Methylen, B Methylen, Z Halogen, Wasserstoff oder $C_1$-$C_4$-Alkyl und n 1 bedeuten.

3. Anilinderivate der Formel I gemäß Anspruch 1, dadurch gekennzeichnet, daß $R^1$ den Rest $-N(CH_3)(OCH_3)$, A Methylen, X Methylen, B Dimethylen, Z Chlor, Wasserstoff oder Methyl und n 1 bedeuten.

4. Verfahren zur Herstellung der Anilinderivate der Formel I gemäß Anspruch 1, dadurch gekennzeichnet, daß man ein Amin der Formel

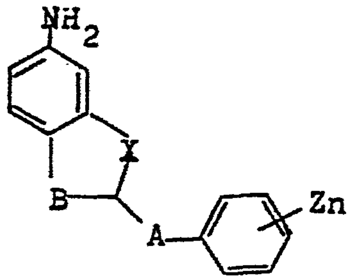 (II),

in der A, B, X, Z und n die im Anspruch 1 genannten Bedeutungen haben,

mit einer Verbindung der Formel

$R^1$-CO-X (III),

in der $R^1$ die im Anspruch 1 angegebenen Bedeutungen hat und X eine Abgangsgruppe oder den Rest $R^1$-CO-O- bedeutet,

in Gegenwart eines inerten organischen Lösungsmittels und eines Säureakzeptors bei einer Temperatur im Bereich zwischen 0 und 80°C umsetzt.

5. Herbizid, enthaltend ein Anilinderivat der Formel I gemäß Anspruch 1.

6. Herbizid, enthaltend inerte Zusatzstoffe und ein Anilinderivat der Formel I gemäß Anspruch 1.

7. Herbizid, enthaltend inerte Zusatzstoffe und 0,1 bis 95 Gew.% eines Anilinderivats der Formel I gemäß Anspruch 1.

8. Verfahren zur Bekämpfung unerwünschten Pflanzenwuchses, dadurch gekennzeichnet, daß man eine herbizid wirksame Menge eines Anilinderivats der Formel I gemäß Anspruch 1 auf die Pflanzen und/oder ihren Standort einwirken läßt.

9. Amine der Formel

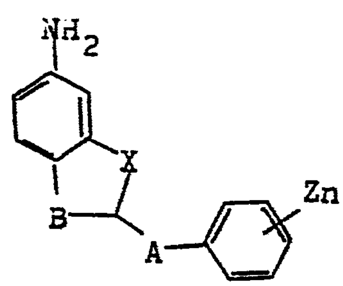 (II),

in der

A eine gegebenenfalls durch $C_1$-$C_5$-Alkyl substituierte Alkylenkette mit 1 bis 5 Kohlenstoffatomen,

B gegebenenfalls durch $C_1$-$C_3$-Alkyl substituiertes Methylen oder Dimethylen,

X $-CH_2-$, $-CH(OH)-$ oder $-CO-$,

Z Wasserstoff, Halogen, $C_1$-$C_6$-Alkyl, $C_1$-$C_6$-Alkoxy, $C_1$-$C_6$-Halogenalkyl oder $C_1$-$C_6$-Halogenalkoxy und

n die Zahlen 1, 2 oder 3 bedeuten.

10. Verwendung von Aminen der Formel II gemäß Anspruch 9 zur Herstellung von Anilinderivaten der Formel I gemäß Anspruch 1.

Europäisches Patentamt

# EUROPÄISCHER RECHERCHENBERICHT

Nummer der Anmeldung

EP 84106722.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | EP - A1 - 0 040 859 (SUMITOMO CHEMICAL COMPANY)<br>* Ansprüche 1,8-10,12 *<br>-- | 1,4-9 | C 07 C 127/19<br>C 07 C 125/065<br>C 07 C 155/02<br>C 07 C 103/34<br>C 07 C 103/737<br>C 07 C 87/64<br>C 07 C 91/28<br>C 07 C 97/10<br>C 07 D 295/20 |
| A | EP - A2 - 0 041 145 (BASF AKTIENGESELLSCHAFT)<br>* Ansprüche 1,2,5 *<br>-- | 1,5-8 | |
| A | DE - A1 -2 718 947 (SCHERICO LTD.)<br>* Anspruch 1 *<br>-- | 1 | |
| P,A | EP - A2 - 0 081 206 (BASF AKTIENGESELLSCHAFT)<br>* Ansprüche 1,5,6,8-10 *<br>-- | 1,4-10 | RECHERCHIERTE SACHGEBIETE (Int. Cl. 3) |
| P,A | DE - A1 - 3 202 624 (BASF AG)<br>* Ansprüche 1,4-8 *<br>-- | 1,4-9 | C 07 C 127/00<br>C 07 C 125/00<br>C 07 C 155/00<br>C 07 C 103/00<br>C 07 C 87/00<br>C 07 C 91/00<br>C 07 C 97/00<br>C 07 D 295/00 |
| A | EP - A1 - 0 041 613 (BASF AKTIENGESELLSCHAFT)<br>* Ansprüche 1-4 *<br>-- | 1,5-8 | |
| A | GB - A - 1 417 322 (CIBA-GEIGY)<br>* Ansprüche 1,10,14-17 *<br>-- | 1,4-9 | |
| A | US - A - 3 757 016 (DON L. HUNTER)<br>* Anspruch 1; Zusammenfassung *<br>-- | 1,5,8 | |

Der vorliegende Recherchenbericht wurde für alle Patentansprüche erstellt.

| Recherchenort | Abschlußdatum der Recherche | Prüfer |
|---|---|---|
| WIEN | 10-09-1984 | REIF |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

Europäisches Patentamt

**0129192**

Nummer der Anmeldung

# EUROPÄISCHER RECHERCHENBERICHT

EP 84106722.6

| EINSCHLÄGIGE DOKUMENTE | | | KLASSIFIKATION DER ANMELDUNG (Int Cl ) |
|---|---|---|---|
| Kategorie | Kennzeichnung des Dokuments mit Angabe, soweit erforderlich, der Maßgeblichen Teile | betrifft Anspruch | |
| A | US - A - 2 913 322 (DAVID J. BEAVER)<br>* Beispiel 1; Ansprüche 3-6 *<br>---- | 1,5,6,<br>8 | |
| | | | RECHERCHIERTE SACHGEBIETE (Int Cl ) |